# EUROPEAN PATENT APPLICATION

(11) **EP 4 400 602 A1**
(43) Date of publication of application: **17.07.2024**
(21) Application number: 21956299.8
(22) Date of filing: 07.09.2021
(51) Int. Cl.: C12Q 1/6869

(54) **METHOD FOR ANALYZING SEQUENCE OF TARGET POLYNUCLEOTIDE**

(71) Applicant: MGI Tech Co., Ltd., Shenzhen, Guangdong 518083 (CN)
(72) Inventor: GONG, Meihua, SHENZHEN, Guangdong 518083 (CN); ZHOU, Shuang, SHENZHEN, Guangdong 518083 (CN); WANG, Jingjing, SHENZHEN, Guangdong 518083 (CN); LI, Jiguang, SHENZHEN, Guangdong 518083 (CN); MA, Kexin, SHENZHEN, Guangdong 518083 (CN); LUO, Yufen, SHENZHEN, Guangdong 518083 (CN); XU, Chongjun, SHENZHEN, Guangdong 518083 (CN); JIANG, Hui, SHENZHEN, Guangdong 518083 (CN); LIU, Jian, SHENZHEN, Guangdong 518083 (CN)
(74) Representative: Weickmann & Weickmann PartmbB
(86) International application number: PCT/CN2021/116930
(87) International publication number: WO 2023/035110

(57) **Abstract**

The present invention relates to a method for analyzing the sequence of a target polynucleotide by polymerizing a nucleotide mixture and a polymerase multiple times to achieve full polymerization, thereby satisfying the requirements of sequencing, and improving read length and accuracy. Further, the present invention also relates to a reagent test kit, the reagent test kit being used for analyzing or sequencing polynucleotides.

## Description

### Technical Field

The present invention relates to a method for analyzing a sequence of a target polynucleotide, which achieves efficient and sufficient polymerization by polymerizing a nucleotide mixture multiple times to meet the needs of sequencing. Furthermore, the present invention also relates to a kit, which can be used to analyze or sequence a polynucleotide.

### Background

High-throughput sequencing, of which sequencing by synthesis (SBS) is the commercial mainstream, mainly uses DNA polymerase and nucleotides with reversible blocking and fluorescent labels to identify DNA sequences. It can sequence hundreds of thousands to millions of DNA molecules in parallel at a time, and has the advantages of high throughput, fast detection speed, flexibility and versatility, and low cost.

Currently, the specific process of sequencing by synthesis comprises employing amplification or rolling circle replication to generate a large number of DNA templates, then anchoring specific sequencing primers, and adding DNA polymerases and fluorescently labeled nucleotides to the reaction system at the same time; or adding DNA polymerases and a mixture of fluorescently labeled nucleotides and non-fluorescently modified nucleotides to the reaction system at the same time. The 3'-OH of these dNTPs is protected, so only one dNTP can be added at a time. Each time a dNTP is added, the replication reaction of the DNA chain will stop, then the fluorescence signal will be excited and collected, and then a chemical reagent will be added to quench the fluorescence signal and the dNTP 3'-OH protecting group will be removed so that the next round sequencing reaction can be carried out.

However, this sequencing technology requires particularly high synthesis efficiency. Incomplete synthesis among multiple copies will cause signal confusion, thereby affecting sequencing accuracy and sequencing read length. This is also one of the key technical issues currently faced by the second-generation sequencing. As people demand shorter sequencing time and greater throughput, the reaction efficiency of polymerases is limited, especially the polymerization ability of nucleotides with fluorescent groups is limited, and these will cause the polymerization efficiency to affect the quality of sequencing. Although the prior art also mentions that polymerization efficiency could be improved by performing simultaneous polymerization of a mixture solution of fluorescently labeled and non-fluorescently modified nucleotides in the reaction system, the effect is still limited. Once these polymerization efficiencies cannot keep up with the sequencing speed, incomplete polymerization would occur in multiple copies, which will lead to signal confusion during sequencing, thus limiting the read length and accuracy of sequencing.

Therefore, there is a need to provide a method for sequencing polynucleotides to improve the read length and accuracy of sequencing.

### Contents of the present invention

The present application was completed by increasing the number of polymerization reactions and adjusting the nucleotide components involved in the polymerization reactions so that the polymerization rate can meet the needs of sequencing and improve the read length and quality of sequencing.

Therefore, in a first aspect, the present application provides a method for analyzing a sequence of target polynucleotide, comprising
(a) providing a target polynucleotide,
(b) contacting the target polynucleotide with a primer under a condition that allows hybridization or annealing, thereby forming a partial duplex comprising the target polynucleotide and the primer used as a growing chain,
(c) contacting the partial duplex with a polymerase and a first nucleotide mixture under a condition that allows the polymerase to perform a nucleotide polymerization reaction such that the growing chain is extended, wherein the first nucleotide mixture comprises at least one nucleotide labeled with a label;
   optionally, the first nucleotide mixture further comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
   wherein each nucleotide in the first nucleotide mixture comprises on its ribose or deoxyribose moiety a protecting group capable of reversibly blocking nucleic acid chain extension (e.g., a protecting group attached via a 2'- or 3'-oxygen atom group);
(d) contacting the product of the previous step with a polymerase and a second nucleotide mixture under a condition that allows the polymerase to perform a nucleotide polymerization reaction such that the growing chain is extended, wherein the second nucleotide mixture comprises at least one (e.g., one, two, three, or four) of: (1) an unlabeled nucleotide, or (2) an irreversible blocking nucleotide, or (3) a combination of the unlabeled nucleotide and irreversible blocking nucleotide;
   in certain embodiments, the second nucleotide mixture comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
   wherein each unlabeled nucleotide in the second nucleotide mixture comprises on its ribose or deoxyribose moiety a protecting group capable of reversibly blocking nucleotide chain extension (e.g., a protecting group attached via a 2'- or 3'-oxygen atom);
(e) not performing this step if at least one nucleotide in the second nucleotide mixture comprises an irreversible blocking nucleotide;
   contacting the product of the previous step with a polymerase and a third nucleotide mixture under a condition that allows the polymerase to perform a nucleotide polymerization reaction such that the growing chain is extended if the second nucleotide mixture does not comprise an irreversible blocking nucleotide; wherein the third nucleotide mixture comprises at least one irreversible blocking nucleotide;
(f) detecting the presence of a label in the product of the previous step;
(g) removing the protective group and label contained in the product of the previous step;
(h) optionally repeating steps (c) to (g) one or more times;
thereby, obtaining a sequence information of the target polynucleotide.

The method for sequencing a sequence of target polynucleotide can be performed as follows: denaturing the sequence of target polynucleotide, contacting the target polynucleotide with different nucleotides respectively to form a complement of the target nucleotide, and detecting the incorporation of the nucleotides. The method utilizes polymerization, allowing the polymerase to extend the growing chain by incorporating the correct nucleotides complementary to the target.

For each round of polymerization, the nucleotide incorporation is performed by the polymerase. Many different polymerases exist, and determining the most suitable polymerase is readily apparent to one of ordinary skill in the art. Preferred enzymes include DNA polymerase I, Klenow fragment, DNA polymerase III, T4 or T7 DNA polymerase, Taq polymerase or vent polymerase. Polymerases engineered to have specific properties can also be used.

The conditions under which polymerization is carried out are well known to those skilled in the art. To carry out the polymerase reaction, a primer is typically first annealed to the target polynucleotide, in which the primer is recognized by the polymerase and serves as the starting site for subsequent extension of the growing chain. The primer may be added as a separate component relative to the target polynucleotide. Other conditions necessary for carrying out the polymerase reaction are well known to those skilled in the art, and these conditions include temperature, pH, and buffer composition.

In certain embodiments, the first nucleotide mixture of the present invention is contacted with the primer and polymerase to enable a first round of polymerization. The nucleotides can be added sequentially (i.e., each type of nucleotide (A, C, G or TAJ) is added separately) or simultaneously. According to the needs of the sequencing rate, the second nucleotide mixture of the present invention is contacted with the polymerase again to perform a second round of polymerization. The nucleotides can be added sequentially (i.e., each type of nucleotide (A, C, G or T/U) is added separately) or simultaneously. Finally, depending on whether the second nucleotide mixture comprises or does not comprise a dideoxynucleotide, a third round of polymerization is either not performed or performed. In certain embodiments, the second nucleotide mixture does not comprise a dideoxynucleotide, and the third nucleotide mixture of the present invention is contacted with the polymerase to enable a third round of polymerization. The nucleotides can be added sequentially (i.e., each type of nucleotide (A, C, G or TAJ) is added separately) or simultaneously.

In certain embodiments, unincorporated nucleotides are removed. For example, unincorporated nucleotides are removed by performing a washing step.

In certain embodiments, the label is detected. The detection can be performed by conventional methods, and means of detecting fluorescent labels or signals are well known in the art. For example, this can be achieved by a device that detects the wavelength of fluorescence. Such devices are well known in the art. For example, such device may be a confocal scanning microscope that scans the surface of a solid support with a laser to image a fluorophore that is directly bound to the nucleic acid molecule being sequenced. Additionally, each signal generated can be observed, for example, with a sensitive 2-D detector, such as a charge-coupled detector (CCD). Other techniques such as scanning near-field optical microscopy (SNOM) may also be used, for example.

In certain embodiments, after the detection, the label can be removed using appropriate conditions.

The use of the labeled nucleotides in the present invention is not limited to DNA sequencing technology. The nucleotides of the present invention can also be used to perform other research forms including polynucleotide synthesis, DNA hybridization analysis, and single nucleotide polymorphism research. Any technology involving interactions between nucleotides and enzymes may utilize the molecules of the present invention. For example, the molecules can be used as a substrate for reverse transcriptase or terminal transferase.

In certain embodiments, in step (c), the extension is an extension using a target polynucleotide as a template. In certain embodiments, the extension is an extension of one nucleotide.

In certain embodiments, the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide. Optionally, the first nucleotide mixture further comprises at least one unlabeled nucleotide.

In certain embodiments, the first nucleotide mixture comprises a first nucleotide labeled with a first label, a second nucleotide labeled with a second label, a third nucleotide labeled with a third label, a fourth nucleotide labeled with a fourth label, an unlabeled first nucleotide, an unlabeled second nucleotide, an unlabeled third nucleotide and an unlabeled fourth nucleotide, or comprises a first nucleotide labeled with a first label, a second nucleotide labeled with a second label, a third nucleotide co-labeled with the first label and the second label, an unlabeled fourth nucleotide, an unlabeled first nucleotide, an unlabeled second nucleotide and an unlabeled third nucleotide.

In certain embodiments, the ratio of the first nucleotide labeled with the first label to the unlabeled first nucleotide is 20:1 to 1:10 (e.g., 10:1 to 1:10, 5:1 to 1:5, 3:1 to 1:3). In certain embodiments, the ratio of the first nucleotide labeled with the first label to the unlabeled first nucleotide is 20:1, 1:10, 1:1, or 3:2. In certain embodiments, the ratio of the first nucleotide labeled with the first label to the unlabeled first nucleotide is 3:2.

In certain embodiments, the ratio of the second nucleotide labeled with the second label to the unlabeled second nucleotide is 20:1 to 1:10 (e.g., 10:1 to 1:10, 5:1 to 1:5, 3:1 to 1:3). In certain embodiments, the ratio of the second nucleotide labeled with the second label to the unlabeled second nucleotide is 20:1, 1:10, 1:1, or 3:2. In certain embodiments, the ratio of the second nucleotide labeled with the second label to the unlabeled second nucleotide is 3:2.

In certain embodiments, the ratio of the third nucleotide labeled with the third label to the unlabeled third nucleotide is 20:1 to 1:10 (e.g., 10:1 to 1:10, 5:1 to 1:5, 3:1 to 1:3). In certain embodiments, the ratio of the third nucleotide labeled with the third label to the unlabeled third nucleotide is 20:1, 1:10, 1:1, or 3:2. In certain embodiments, the ratio of the third nucleotide labeled with the third label to the unlabeled third nucleotide is 3:2.

In certain embodiments, the ratio of the third nucleotide co-labeled with the first label and the second label to the unlabeled third nucleotide is 20:1 to 1:10 (e.g., 10:1 to 1:10, 5:1 to 1:5, 3:1 to 1:3). In certain embodiments, the ratio of the third nucleotide co-labeled with the first label and the second label to the unlabeled third nucleotide is 20:1, 1:10, 1: 1 or 3:2. In certain embodiments, the ratio of the third nucleotide co-labeled with the first label and the second label to the unlabeled third nucleotide is 3:2.

In certain embodiments, the ratio of the fourth nucleotide labeled with the fourth label to the unlabeled fourth nucleotide is 20:1 to 1:10 (e.g., 10:1 to 1:10, 5:1 to 1:5, 3:1 to 1:3). In certain embodiments, the ratio of the fourth nucleotide labeled with the fourth label to the unlabeled fourth nucleotide is 20:1, 1:10, 1:1, or 3:2. In certain embodiments, the ratio of the fourth nucleotide labeled with the fourth label to the unlabeled fourth nucleotide is 3:2.

In certain embodiments, in step (d), the second nucleotide mixture comprises:
(1) an unlabeled first nucleotide, an unlabeled second nucleotide, an unlabeled third nucleotide, and an unlabeled fourth nucleotide; or,
(2) a first irreversible blocking nucleotide, a second irreversible blocking nucleotide, a third irreversible blocking nucleotide, and a fourth irreversible blocking nucleotide; or,
(3) comprising an unlabeled first nucleotide, an unlabeled second nucleotide, an unlabeled third nucleotide, an unlabeled fourth nucleotide, and a first irreversible blocking nucleotide, a second irreversible blocking nucleotide, a third irreversible blocking nucleotide, and a fourth irreversible blocking nucleotide.

In certain embodiments, the ratio of the unlabeled first nucleotide to the first irreversible blocking nucleotide is 100:1 to 1:100, or there is only the irreversible blocking nucleotide. In certain embodiments, the ratio of the unlabeled first nucleotide to the first irreversible blocking nucleotide is 1:100, 50:1, or 100:1. In certain embodiments, the ratio of the unlabeled first nucleotide to the first irreversible blocking nucleotide is 100:1.

In certain embodiments, the ratio of the unlabeled second nucleotide to the second irreversible blocking nucleotide is 100:1 to 1:100, or there is only the irreversible blocking nucleotide. In certain embodiments, the ratio of the unlabeled second nucleotide to the second irreversible blocking nucleotide is 1:100, 50:1, or 100:1. In certain embodiments, the ratio of the unlabeled second nucleotide to the second irreversible blocking nucleotide is 100:1.

In certain embodiments, the ratio of the unlabeled third nucleotide to the third irreversible blocking nucleotide is 100:1 to 1:100, or there is only irreversible blocking nucleotide. In certain embodiments, the ratio of the unlabeled third nucleotide to the third irreversible blocking nucleotide is 1:100, 50:1, or 100:1. In certain embodiments, the ratio of the unlabeled third nucleotide to the third irreversible blocking nucleotide is 100:1.

In certain embodiments, the ratio of the unlabeled fourth nucleotide to the fourth irreversible blocking nucleotide is 100:1 to 1:100, or there is only the irreversible blocking nucleotide. In certain embodiments, the ratio of the unlabeled fourth nucleotide to the fourth irreversible blocking nucleotide is 1:100, 50:1, or 100:1. In certain embodiments, the ratio of the unlabeled fourth nucleotide to the fourth irreversible blocking nucleotide is 100:1.

In certain embodiments, the first label, the second label, the third label and the fourth label are each independently the same or different.

In certain embodiments, the first label, the second label, the third label and the fourth label are different.

In certain embodiments, the first label, the second label, the third label and the fourth label are luminescent labels (e.g., fluorescent labels).

In certain embodiments, the first label, the second label, the third label and the fourth label are each independently selected from the group consisting of coumarin, AlexaFluor, Bodipy, fluorescein, tetramethylrhodamine, Phenoxazine, acridine, Cy5, Cy3, AF532, Texas Red and derivative thereof.

In certain embodiments, the target polynucleotide comprises or is DNA, RNA, or any combination thereof. In certain embodiments, the extension product of the nucleic acid molecule is DNA.

In certain embodiments, the target polynucleotide is obtained from a sample derived from eukaryote (e.g., animal, plant, fungus), prokaryote (e.g., bacterium, actinomycete), virus, phage, or any combination thereof.

In certain embodiments, the first nucleotide, the second nucleotide, the third nucleotide, and the fourth nucleotide are each independently selected from the group consisting of A, T, C, G, and U.

In certain embodiments, the first nucleotide, the second nucleotide, the third nucleotide, and the fourth nucleotide are each different.

In certain embodiments, the first nucleotide, the second nucleotide, the third nucleotide, and the fourth nucleotide are A, T, C, G, respectively. In certain embodiments, the first irreversible blocking nucleotide, the second irreversible blocking nucleotide, the third irreversible blocking nucleotide and the fourth irreversible blocking nucleotide are each independently selected from the group consisting of A, T, C, G, U.

In certain embodiments, the first irreversible blocking nucleotide, the second irreversible blocking nucleotide, the third irreversible blocking nucleotide, and the fourth irreversible blocking nucleotide are different.

In certain embodiments, the first irreversible blocking nucleotide, the second irreversible blocking nucleotide, the third irreversible blocking nucleotide and the fourth irreversible blocking nucleotide are A, T, C, G, respectively.

In certain embodiments, the irreversible blocking nucleotide is a dideoxynucleotide.

In certain embodiments, when the first nucleotide mixture comprises a first nucleotide labeled with a first label, a second nucleotide labeled with a second label, a third nucleotide labeled with a third label, and a fourth nucleotide labeled with a fourth label; the second nucleotide mixture comprises an unlabeled first nucleotide, an unlabeled second nucleotide, an unlabeled third nucleotide, and an unlabeled fourth nucleotide.

In certain embodiments, when the first nucleotide mixture comprises a first nucleotide labeled with a first label, a second nucleotide labeled with a second label, a third nucleotide labeled with a third label nucleotide and an unlabeled fourth nucleotide, or comprises a first nucleotide labeled with a first label, a second nucleotide labeled with a second label, a third nucleotide co-labeled with the first label and the second label, and an unlabeled fourth nucleotide, the second nucleotide mixture comprises an unlabeled first nucleotide, an unlabeled second nucleotide, and an unlabeled third nucleotide.

In a second aspect, the present application provides a kit, which comprises:
(a) a first nucleotide mixture, which comprises at least one nucleotide labeled with a label;
   optionally, the first nucleotide mixture further comprises at least one unlabeled nucleotide; for example, comprises a nucleotide selected from the group consisting of an unlabeled first nucleotide, an unlabeled second nucleotide, an unlabeled third nucleotide, an unlabeled fourth nucleotide, or any combination thereof;
   wherein each nucleotide in the first nucleotide mixture comprises on its ribose or deoxyribose moiety a protecting group capable of reversibly blocking nucleic acid chain extension (e.g., a protecting group attached via a 2'- or 3'-oxygen atom);
(b) a second nucleotide mixture, which comprises at least one (e.g., one, two, three or four) of (1) an unlabeled nucleotide, or (2) an irreversible blocking nucleotide, or (3) a combination of the unlabeled nucleotide and irreversible blocking nucleotide;

In certain embodiments, the second nucleotide mixture comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof.
wherein each unlabeled nucleotide in the second nucleotide mixture comprises on its ribose or deoxyribose moiety a protecting group capable of reversibly blocking nucleic acid chain extension (e.g., a protecting group attached via a 2'- or 3'-oxygen atom);
(c) if the second nucleotide mixture does not comprise an irreversible blocking nucleotide, the kit comprises a third nucleotide mixture which comprises at least one irreversibly blocking nucleotide;
if at least one nucleotide in the second nucleotide mixture comprises an irreversible blocking nucleotide, the kit does not comprise a third nucleotide mixture.

In certain embodiments, the second nucleotide mixture comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof.

In certain embodiments, the irreversible blocking nucleotide is a dideoxynucleotide.

In certain embodiments, the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide. Optionally, the first nucleotide mixture further comprises at least one unlabeled nucleotide.

In certain embodiments, the first nucleotide mixture comprises a first nucleotide labeled with a first label, a second nucleotide labeled with a second label, a third nucleotide labeled with a third label, a fourth nucleotide labeled with a fourth label, an unlabeled first nucleotide, an unlabeled second nucleotide, an unlabeled third nucleotide and an unlabeled fourth nucleotide, or comprises a first nucleotide labeled with a first label, a second nucleotide labeled with a second label, a third nucleotide co-labeled with the first label and the second label, an unlabeled fourth nucleotide, an unlabeled first nucleotide, an unlabeled second nucleotide and an unlabeled third nucleotide.

In certain embodiments, the ratio of the first nucleotide labeled with the first label to the unlabeled first nucleotide is 20:1 to 1:10 (e.g., 10:1 to 1:10, 5:1 to 1:5, 3:1 to 1:3). In certain embodiments, the ratio of the first nucleotide labeled with the first label to the unlabeled first nucleotide is 20:1, 1:10, 1:1, or 3:2. In certain embodiments, the ratio of the first nucleotide labeled with the first label to the unlabeled first nucleotide is 3:2.

In certain embodiments, the ratio of the second nucleotide labeled with the second label to the unlabeled second nucleotide is 20:1 to 1:10 (e.g., 10:1 to 1:10, 5:1 to 1:5, 3:1 to 1:3). In certain embodiments, the ratio of the second nucleotide labeled with the second label to the unlabeled second nucleotide is 20:1, 1:10, 1:1, or 3:2. In certain embodiments, the ratio of the second nucleotide labeled with the second label to the unlabeled second nucleotide is 3:2.

In certain embodiments, the ratio of the third nucleotide labeled with the third label to the unlabeled third nucleotide is 20:1 to 1:10 (e.g., 10:1 to 1:10, 5:1 to 1:5, 3:1 to 1:3). In certain embodiments, the ratio of the third nucleotide labeled with the third label to the unlabeled third nucleotide is 20:1, 1:10, 1:1, or 3:2. In certain embodiments, the ratio of the third nucleotide labeled with the third label to the unlabeled third nucleotide is 3:2.

In certain embodiments, the ratio of the third nucleotide co-labeled with the first label and the second label to the unlabeled third nucleotide is 20:1 to 1:10 (e.g., 10:1 to 1:10, 5:1 to 1:5, 3:1 to 1:3). In certain embodiments, the ratio of the third nucleotide co-labeled with the first label and the second label to the unlabeled third nucleotide is 20:1,1:10, 1:1 or 3:2. In certain embodiments, the ratio of the third nucleotide co-labeled with the first label and the second label to the unlabeled third nucleotide is 3:2.

In certain embodiments, the ratio of the fourth nucleotide labeled with the fourth label to the unlabeled fourth nucleotide is 20:1 to 1:10 (e.g., 10:1 to 1:10, 5:1 to 1:5, 3:1 to 1:3). In certain embodiments, the ratio of the fourth nucleotide labeled with the fourth label to the unlabeled fourth nucleotide is 20:1, 1:10, 1:1, or 3:2. In certain embodiments, the ratio of the fourth nucleotide labeled with the fourth label to the unlabeled fourth nucleotide is 3:2.

In certain embodiments, when the first nucleotide mixture comprises a first nucleotide labeled with a first label, a second nucleotide labeled with a second label, a third nucleotide labeled with a third label and a fourth nucleotide labeled with a fourth label; the second nucleotide mixture comprises an unlabeled first nucleotide, an unlabeled second nucleotide, an unlabeled third nucleotide, an unlabeled fourth nucleotide.

In certain embodiments, when the first nucleotide mixture comprises a first nucleotide labeled with a first label, a second nucleotide labeled with a second label, a third nucleotide labeled with a third label and an unlabeled fourth nucleotide, or comprises a first nucleotide labeled with a first label, a second nucleotide labeled with a second label, a third nucleotide co-labeled with the first label and the second label and an unlabeled fourth nucleotide, the second nucleotide mixture comprises an unlabeled first nucleotide, an unlabeled second nucleotide and an unlabeled third nucleotide.

In certain embodiments, the second nucleotide mixture comprises:
(1) an unlabeled first nucleotide, an unlabeled second nucleotide, an unlabeled third nucleotide, and an unlabeled fourth nucleotide; or,
(2) a first irreversible blocking nucleotide, a second irreversible blocking nucleotide, a third irreversible blocking nucleotide, and a fourth irreversible blocking nucleotide; or,
(3) an unlabeled first nucleotide, an unlabeled second nucleotide, an unlabeled third nucleotide, an unlabeled fourth nucleotide, and a first irreversible blocking nucleotide, a second irreversible blocking nucleotide, a third irreversible blocking nucleotide, and a fourth irreversible blocking nucleotide.

In certain embodiments, the ratio of the unlabeled first nucleotide to the first irreversible blocking nucleotide is 100:1 to 1:100 or there is only the irreversible blocking nucleotide. In certain embodiments, the ratio of the unlabeled first nucleotide to the first irreversible blocking nucleotide is 1:100, 50:1, or 100:1. In certain embodiments, the ratio of the unlabeled first nucleotide to the first irreversible blocking nucleotide is 100:1.

In certain embodiments, the ratio of the unlabeled second nucleotide to the second irreversible blocking nucleotide is 100:1 to 1:100, or there is only the irreversible blocking nucleotide. In certain embodiments, the ratio of the unlabeled second nucleotide to the second irreversible blocking nucleotide is 1:100, 50:1, or 100:1. In certain embodiments, the ratio of the unlabeled second nucleotide to the second irreversible blocking nucleotide is 100:1.

In certain embodiments, the ratio of the unlabeled third nucleotide to the third irreversible blocking nucleotide is 100:1 to 1:100, or there is only the irreversible blocking nucleotide. In certain embodiments, the ratio of the unlabeled third nucleotide to the third irreversible blocking nucleotide is 1:100, 50:1, or 100:1. In certain embodiments, the ratio of the unlabeled third nucleotide to the third irreversible blocking nucleotide is 100:1.

In certain embodiments, the ratio of the unlabeled fourth nucleotide to the fourth irreversible blocking nucleotide is 100:1 to 1:100, or there is only the irreversible blocking nucleotide. In certain embodiments, the ratio of the unlabeled fourth nucleotide to the fourth irreversible blocking nucleotide is 1:100, 50:1, or 100:1. In certain embodiments, the ratio of the unlabeled fourth nucleotide to the fourth irreversible blocking nucleotide is 100:1.

In certain embodiments, the irreversible blocking nucleotide is a dideoxynucleotide.

In certain embodiments, the kit further comprises one or more selected from the group consisting of a nucleic acid polymerase, a reagent for performing extension, a reagent for performing sequencing, or any combination thereof.

In certain embodiments, the reagent for performing extension comprises one or more selected from the group consisting of a primer complementary to all or part of the polynucleotide, a DNB preparation buffer, a working buffer for enzyme (e.g., nucleic acid polymerization enzyme), water, a solution containing ions (e.g., Mg²⁺), a single-stranded DNA binding protein, or any combination thereof.

In certain embodiments, the reagent for sequencing comprises one or more selected from the group consisting of: a sequencing slide, a reagent for removing protective group and label on nucleotide, a reagent for detecting a luminescent signal of the label (e.g., a fluorescent mixture solution).

In certain embodiments, the nucleic acid polymerase is a DNA polymerase, such as a thermostable DNA polymerase. In certain embodiments, the thermostable DNA polymerase is obtained from, *Thermus aquaticus (Taq), Thermus thermophiles (Tth), Thermus filiformis, Thermis flavus, Thermococcus literalis, Thermus antranildanii, Thermus caldophllus, Thermus chliarophilus, Thermus flavus, Thermus igniterrae, Thermus lacteus, Thermus oshimai, Thermus ruber, Thermus rubens, Thermus scotoductus, Thermus silvanus, Thermus thermophllus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus woesei, Pyrococcus horikoshii, Pyr ococcus abyssi, Pyrodictium occultum, Aquifexpyrophilus* and *Aquifex aeolieus.*

In certain embodiments, the kit is used for analyzing a polynucleotide.

In certain embodiments, the kit is used for sequencing a polynucleotide.

In certain embodiments, the presence of the label is detected by a luminescent signal.

In certain embodiments, the presence of the label is detected by one or more (e.g., 2, 3, 4) luminescent signals.

In certain embodiments, the first label, the second label, the third label and the fourth label are each independently the same or different.

In certain embodiments, the first label, the second label, the third label and the fourth label are different.

In certain embodiments, the first label, the second label, the third label and the fourth label are luminescent labels (e.g., fluorescent labels).

In certain embodiments, the first label, the second label, the third label and the fourth label are each independently selected from the group consisting of coumarin, AlexaFluor, Bodipy, fluorescein, tetramethylrhodamine, Phenoxazine, acridine, Cy5, Cy3, AF532, EF700, Texas Red and derivative thereof.

In certain embodiments, the target polynucleotide comprises or is DNA, RNA, or any combination thereof. In certain embodiments, the extension product of the nucleic acid molecule is DNA.

In certain embodiments, the target polynucleotide is obtained from a sample derived from eukaryote (e.g., animal, plant, fungus), prokaryote (e.g., bacterium, actinomycete), virus, phage, or any combination thereof.

In certain embodiments, the first nucleotide, the second nucleotide, the third nucleotide, and the fourth nucleotide are each independently selected from the group consisting of A, T, C, G, and U.

In certain embodiments, the first nucleotide, the second nucleotide, the third nucleotide, and the fourth nucleotide are each different.

In certain embodiments, the first nucleotide, the second nucleotide, the third nucleotide, and the fourth nucleotide are A, T, C, G, respectively. In certain embodiments, the first irreversible blocking nucleotide, the second irreversible blocking nucleotide, the third irreversible blocking nucleotide and the fourth irreversible blocking nucleotide are each independently selected from the group consisting of A, T, C, G, U.

In certain embodiments, the first irreversible blocking nucleotide, the second irreversible blocking nucleotide, the third irreversible blocking nucleotide, and the fourth irreversible blocking nucleotide are different.

In certain embodiments, the first irreversible blocking nucleotide, the second irreversible blocking nucleotide, the third irreversible blocking nucleotide and the fourth irreversible blocking nucleotide are A, T, C, G, respectively.

In certain embodiments, the irreversible blocking nucleotide is a dideoxynucleotide.

### Definition of Terms

Unless otherwise defined, all technical and scientific terms used herein have the same meaning as commonly understood by one of ordinary skill in the art to which this invention belongs. All patents, applications, and other publications mentioned herein are incorporated by reference in their entirety. To the extent that definitions set forth herein conflict or are inconsistent with definitions set forth in patents, applications, and other publications incorporated herein by reference, the definitions set forth herein shall prevail.

As used herein, the term "polynucleotide" refers to a deoxyribonucleic acid (DNA), ribonucleic acid (RNA), or the like. Polynucleotides can be single-stranded, double-stranded, or contain both single- and double-stranded sequences. Polynucleotide molecules can be derived from double-stranded DNA (dsDNA) forms (e.g., genomic DNA, PCR and amplification products, etc.), or can be derived from single-stranded forms of DNA (ssDNA) or RNA and can be converted into dsDNA forms, and vice versa. The exact sequence of a polynucleotide molecule may be known or unknown. The followings are illustrative examples of polynucleotides: gene or gene fragment (e.g., probe, primer, EST or SAGE tag), genomic DNA, genomic DNA fragment, exon, intron, messenger RNA (mRNA), transporter RNA, ribosomal RNA, ribozyme, cDNA, recombinant polynucleotide, synthetic polynucleotide, branched polynucleotide, plasmid, vector, isolated DNA of any sequence, isolated RNA of any sequence, and nucleic acid probe, primer or amplification copy of any of the foregoing sequences.

Polynucleotides may include nucleotides or nucleotide analogs. Nucleotides usually contain a saccharide (e.g., ribose or deoxyribose), a base, and at least one phosphate group. Nucleotides can be abasic (i.e., lacking bases). Nucleotides include deoxyribonucleotides, modified deoxyribonucleotides, ribonucleotides, modified ribonucleotides, peptide nucleotides, modified peptide nucleotides, modified sugar-phosphate backbone nucleotides and mixtures thereof. Examples of nucleotides include, for example, adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine triphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP) and deoxyuridine triphosphate (dUTP). Nucleotide analogs containing modified bases may also be used in the methods described herein. Exemplary modified bases that may be contained in polynucleotides, whether with a native backbone or similar structures, include, for example, inosine, xathanine, hypoxathanine, isocytosine, isoguanine, 2-aminopurine, 5-methylcytosine, 5-hydroxymethylcytosine, 2-aminoadenine, 6-methyladenine, 6-methylguanine, 2-propylguanine, 2-propyladenine, 2-thiouracil, 2-thiothymine, 2-thiocytosine, 15-halogenated uracil, 15-halogenated cytosine, 5-propynyluracil, 5-propynylcytosine, 6-azouracil, 6-azocytosine, 6-azothymine, 5-uracil, 4-thiouracil, 8-halogenated adenine or guanine, 8-aminoadenine or guanine, 8-thioadenine or guanine, 8-thioalkyladenine or guanine, 8-hydroxyadenine or guanine, 5-halogenated uracil or cytosine, 7-methylguanine, 7-methyladenine, 8-azaguanine, 8-azaadenine, 7-deazaguanine, 7-deazaadenine, 3-deazaguanine, 3-deazaadenine, etc. As is known in the art, certain nucleotide analogs cannot be incorporated into polynucleotides, for example, nucleotide analogs such as adenosine 5'-phosphoryl sulfate.

In general, nucleotides include nucleotides A, C, G, T or U. As used herein, the term "nucleotide A" refers to a nucleotide containing adenine (A) or a modification or analog thereof, such as ATP, dATP. "Nucleotide G" refers to a nucleotide containing guanine (G) or a modification or analog thereof, such as GTP, dGTP. "Nucleotide C" refers to a nucleotide containing cytosine (C) or a modification or analog thereof, such as CTP, dCTP. "Nucleotide T" refers to a nucleotide containing thymine (T) or a modification or analog thereof, such as TTP, dTTP. "Nucleotide U" refers to a nucleotide containing uracil (U) or a modification or analog thereof, such as UTP, dUTP.

As used herein, the term "dideoxynucleotide" refers to a nucleotide with deoxygenation on the 2'- and 3'-carbons of ribose, also known as 2',3'-dideoxynucleotide. Generally, dideoxynucleotide include dideoxynucleotide A, C, G, T or U. The term "dideoxynucleotide A" refers to a dideoxynucleotide containing adenine (A) or analog thereof, such as ddATP. "Dideoxynucleotide G" refers to a dideoxynucleotide containing guanine (G) or analog thereof, such as ddGTP. "Dideoxynucleotide C" refers to a dideoxynucleotide containing cytosine (C) or analog thereof, such as ddCTP. "Dideoxynucleotide T" refers to a dideoxynucleotide containing thymine (T) or analog thereof, such as ddTP. "Dideoxynucleotide U" refers to a dideoxynucleotide containing uracil (U) or analog thereof, such as ddUTP. As used herein, ddNTP refers to one of dideoxyadenosine triphosphate (ddATP), dideoxyguanosine triphosphate (ddGTP), dideoxycytidine triphosphate (ddCTP), dideoxyuridine triphosphate (ddUTP), dideoxythymidine triphosphate (ddTTP) or a combination of two or more thereof, in which dideoxyuridine triphosphate and dideoxythymidine triphosphate do not appear at the same time.

As used herein, the term "label" refers to a group capable of emitting a luminescent signal under certain conditions.

As used herein, the term "luminescent label" refers to any substance capable of emitting fluorescence at a specific emission wavelength when excited by a suitable excitation wavelength. Such luminescent label may be a chemiluminescent label, for example, selected from biochemiluminescent labels that trigger different luminescence kinetics, and any combination thereof, for example, the chemiluminescent label is selected from luciferases that trigger different luminescence kinetics or any combinaiton thereof; such luminescent label may be, for example, a fluorophore selected from coumarin, AlexaFluor, Bodipy, fluorescein, tetramethylrhodamine, phenoxazine, acridine, Cy5, Cy3, EF700, AF532, Texas Red and derivative thereof, etc.

As used herein, the term "protecting group" refers to a group that prevents a polymerase (which incorporates a nucleotide containing the group into a polynucleotide chain being synthesized) to continuously catalyze the incorporation of another nucleotide after the nucleotide containing the group is incorporated into the polynucleotide chain being synthesized. Such protecting group is also referred to herein as 3'-OH protecting group. Nucleotides containing such protecting group are also referred to herein as 3'-blocked nucleotides. A protecting group can be any suitable group that can be added to a nucleotide as long as the protecting group prevents another nucleotide molecule from being added to the polynucleotide chain, and is easily removed from the sugar portion of the nucleotide without damaging the polynucleotide chain. Furthermore, nucleotides modified with protecting groups need to be resistant to polymerases or other suitable enzymes used to incorporate the modified nucleotides into the polynucleotide chain. Therefore, ideal protecting groups exhibit long-term stability, can be efficiently incorporated by polymerases, prevent secondary incorporation or further incorporation of nucleotides, and can be removed under mild conditions, preferably aqueous conditions, without damaging the polynucleotide structure.

The prior art has described a variety of protecting groups conforming to the above description. For example, WO91/06678 discloses that 3'-OH protecting groups include ester and ether, -F, - NH₂, -OCH₃, -N₃, -OPO₃, -NHCOCH₃, 2-nitrobenzene carbonate, 2,4-sulfenyldinitro and tetrahydrofuran ether. Metzker et al. (Nucleic Acids Research, 22(20):4259-4267, 1994) disclose the synthesis and application of eight 3'-modified 2-deoxyribonucleoside 5'-triphosphates (3'-modified dNTPs). WO2002/029003 describes the use of allyl protecting groups to cap 3'-OH groups on growing DNA chains in the polymerase reaction. Preferably, various protecting groups reported in International Application Publications WO2014139596 and WO2004/018497 may be used, including for example those exemplary protecting groups in Fig. 1A and those 3'-hydroxyl protecting groups (i.e., protecting groups) specified in the claims of WO2014139596, and for example those protecting groups illustrated in Fig. 3 and 4 and those protecting groups specified in the claims of WO2004/018497. The above references are incorporated by reference in their entirety.

As used herein, the term "reversible blocking group" refers to a group that, when incorporated into a polynucleotide chain being synthesized, renders the polymerase unable to proceed to the next round of polymerization, thereby causing the termination of polymerization reaction. In this case, in each round of polymerization reaction, one and only one base is incorporated into the growing nucleic acid chain. In addition, this group can be removed, and subsequently, the growing nucleic acid chain can undergo the next round of polymerization, and a base is introduced again. Examples of reversible blocking groups are that the H in 3'-OH is substituted with the following groups: ester, ether, -F, -NH₂, -OCH₃, -N₃, -OPO₃, -NHCOCH₃, 2-nitrobenzene carbonate, 2,4-sulfenyldinitro and tetrahydrofuran ether-CH₂-CH=CH₂, S-S, or that the base is blocked with a large steric hindrance group and a fluorescent group and linked to fluorescence via S-S.

As used herein, the term "irreversible blocking nucleotide" refers to a nucleotide that, upon incorporation into a polynucleotide chain being synthesized, prevents the subsequent incorporation of subsequent nucleotides into the polynucleotide chain due to its blocking effect. And this blocking effect is irreversible. The irreversible blocking nucleotides usually include dideoxynucleotides, or nucleotides in which the 3'-OH is replaced by a methoxy group at the 3' end (3'-OMe, or 3'-OCH₃), and nucleotides with 3'-end azide (3'-N₃) and 3'-end ethoxy (3'-OEt, 3'-OCH₂CH₃).

### Beneficial technical effects of the present invention

In sequencing, the present invention achieves a more efficient polymerization reaction by increasing the number of polymerization reactions, so that the polymerization rate can meet the needs of sequencing and improve the read length and quality of sequencing. Furthermore, the present invention shortens the first round of polymerization and quickly performs the second or the second and third rounds of polymerization while adjusting the nucleotide components participating in the polymerization (e.g., polymerizing nucleotide components without fluorescent labels) to achieve a more efficient polymerization reaction.

The method of the present invention can not only increase the efficiency of the polymerization reaction, but also ensure that the polymerization reaction is sufficient to improve the read length and quality of sequencing. It also reduces signal interference caused by unclean fluorescence excision or unclean elution of the excised fluorescence, thereby improving the excision efficiency, and reducing the sequencing error rates.

### Brief Description of the Drawings

Fig. 1 shows the flow chart of synthesis and sequencing of *E. coli* DNA in Experimental Group 1.
Fig. 2 shows the flow chart of synthesis and sequencing of *E. coli* DNA in Experimental Group 2.
Fig. 3 shows the flow chart of synthesis and sequencing of *E. coli* DNA in Experimental Group 3.
Fig. 4 shows the flow chart of synthesis and sequencing of *E. coli* DNA in Experimental Group 4.
Fig. 5 shows the flow chart of synthesis and sequencing of *E. coli* DNA in Experimental Groups 5, 6 and 7.
Fig. 6 shows the flow chart of synthesis and sequencing of *E. coli* DNA in Experimental Groups 8, 9, and 10.
Fig. 7 shows the Q30 (%) proportion results for each cycle of four experiments, i.e., Experimental Groups 1 to 4. Among them, the abscissa is the number of sequencing cycles, and the ordinate is the Q30 (%) proportion for each cycle.
Fig. 8 shows the sequencing error rate (%) results for each cycle of four experiments, i.e., Experimental Groups 1 to 4. Among them, the abscissa is the number of sequencing cycles, and the ordinate is the sequencing error rate (%) for each cycle.

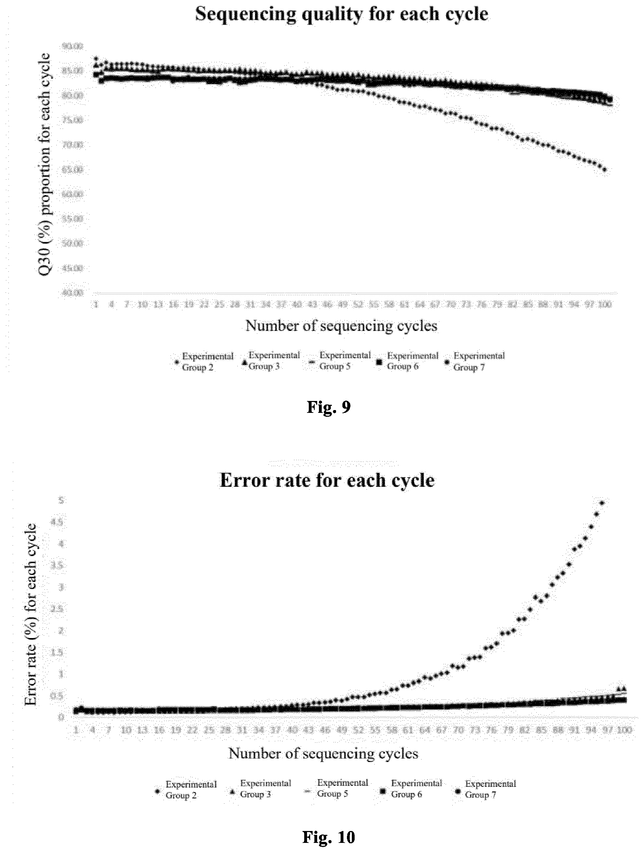

Fig. 9 shows the Q30 (%) proportion results for each cycle of five experiments, i.e., Experimental Groups 2, 3, 5, 6, and 7. Taking Experimental Group 2 as the control, in other experimental groups, 2 rounds of polymerization were performed in each cycle of reaction, in which the nucleotides polymerized in the first round were a mixture prepared with Nucleotide Mixture Solution 1 and Nucleotide Mixture Solution 2 in a ratio of 3:2, and those in the second round were mixtures prepared with Nucleotide Mixture Solution 2 and the dideoxynucleotide mixture solution in different ratios. Among them, the abscissa is the number of sequencing cycles, and the ordinate is the Q30 (%) proportion for each cycle.
Fig. 10 shows the sequencing error rate (%) results for each cycle of five experiments, i.e., Experimental Groups 2, 3, 5, 6, and 7. Taking Experimental Group 2 as the control, in other experimental groups, 2 rounds of polymerization were performed in each cycle of reaction, in which the nucleotides polymerized in the first round were a mixture prepared with Nucleotide Mixture Solution 1 and Nucleotide Mixture Solution 2 in a ratio of 3:2, and those in the second round were mixtures prepared with Nucleotide Mixture Solution 2 and the dideoxynucleotide mixture solution in different ratios. Among them, the abscissa is the number of sequencing cycles, and the ordinate is the sequencing error rate (%) for each cycle.

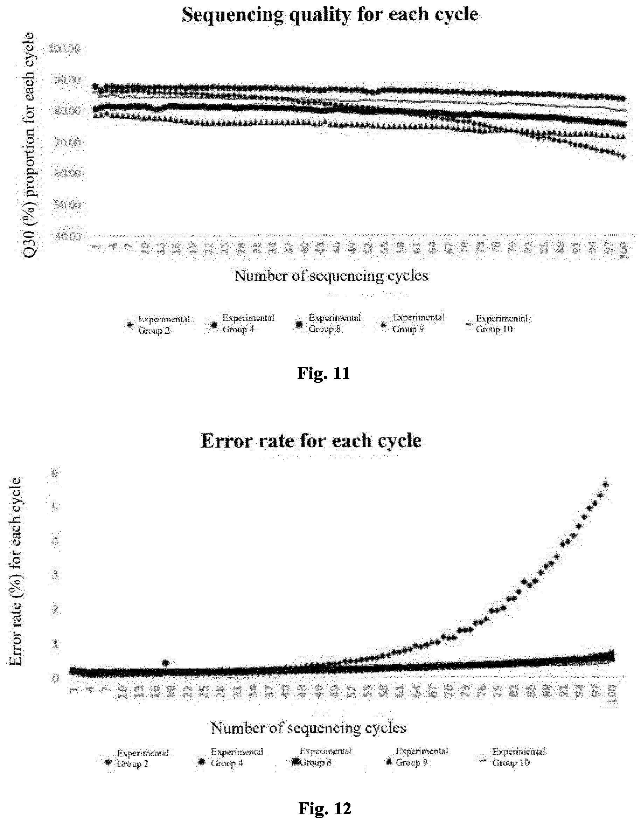

Fig. 11 shows the Q30 (%) proportion results for each cycle of five experiments, i.e., Experimental Groups 2, 4, 8, 9, and 10. Taking Experimental Group 2 as the control, in all other experimental groups, 3 rounds of polymerization were performed in each cycle of reaction, in which the nucleotides polymerized in the first round were mixtures prepared with Nucleotide Mixture Solution 1 and Nucleotide Mixture Solution 2 with different concentrations, those in the second round were Nucleotide Mixture Solution 2, and those in the third round were a the dideoxynucleotide mixture solution. Among them, the abscissa is the number of sequencing cycles, and the ordinate is the Q30 (%) proportion for each cycle.
Fig. 12 shows the sequencing error rate (%) results for each cycle of five experiments, i.e., Experimental Groups 2, 4, 8, 9, and 10. Taking Experimental Group 2 as the control, in all other experimental groups, 3 rounds of polymerization were preformed in each cycle of reaction, in which the nucleotides polymerized in the first round were mixtures prepared with Nucleotide Mixture Solution 1 and Nucleotide Mixture Solution 2 with different concentrations, those in the second round were Nucleotide Mixture Solution 2, and those in the third round were a the dideoxynucleotide mixture solution. Among them, the abscissa is the number of sequencing cycles, and the ordinate is the sequencing error rate (%) for each cycle.

### Specific Models for Carrying Out the present invention

The present invention will now be described with reference to the following examples which are intended to illustrate but not to limit the present invention.

Unless otherwise specified, the molecular biology experimental methods used in the present invention basically refer to J. Sambrook et al., Molecular Cloning: Laboratory Manual, 2nd Edition, Cold Spring Harbor Laboratory Press, 1989, and F. M. Ausubel et al., Compiled Experimental Guide to Molecular Biology, 3rd Edition, John Wiley & Sons, Inc., 1995. Those skilled in the art will appreciate that the examples describe the present invention by way of example and are not intended to limit the scope sought to be protected by the present invention.
1. Key equipment used in this example:
MGISEQ-2000RS sequencer, MGIDL-200H loader, MGISEQ-2000RS sequencing slide.
2. Key reagents used in this example are shown in Table 1 below:

**Table 1. Reagents used**

| Reagent name | Brand | Item No. |
|---|---|---|
| Cold dATP | BGI | 01CATP000-10ml |
| Cold dTTP | BGI | 01CTTP000-10ml |
| Cold dGTP | BGI | 01CGTP000-10ml |
| Cold dCTP | BGI | 01CCTP000-10ml |
| ddATP | BGI | 01DATP000-1ml |
| ddTTP | BGI | 01DTTP000-1ml |
| ddGTP | BGI | 01DGTP000-1ml |
| ddCTP | BGI | 01DCTP000-1ml |

### Example 1

In this example, various reagents were prepared in advance.

### 1) Preparation of Nucleotide Mixture Solution 1

As shown in Table 2 below, all nucleotides in Nucleotide Mixture Solution 1 had both fluorescent dyes and reversible blocking groups, in which A, T, G, and C were adenine nucleotide, thymine nucleotide, guanine nucleotide, and cytosine nucleotide, respectively, and the fluorescent dyes carried by the nucleotides were: dATP-CyS, dGTP-Cy3, dTTP-ROX, and dCTP-EF700, and all of which had reversible blocking groups.

**Table 2: Nucleotide Mixture Solution 1**

| Reagent name | Final concentration (nmol/L) |
|---|---|
| dATP-1 | 200 |
| dTTP-1 | 200 |
| dGTP-1 | 200 |
| dCTP-1 | 200 |

Among them, dATP-1 referred to adenine nucleotide with both reversible blocking group modification and Cy5 fluorescence modification, dTTP-1 referred to thymine nucleotide with both reversible blocking group modification and ROX fluorescence modification, dGTP-1 referred to guanine nucleotide with both reversible blocking group modification and Cy3 fluorescence modification, and dCTP-1 referred to cytosine nucleoside with both reversible blocking group modification and EF700 fluorescence modification.

### 2) Preparation of Nucleotide Mixture Solution 2

As shown in Table 3 below, all nucleotides in Nucleotide Mixture Solution 2 only had reversible blocking groups, in which A, T, G, and C were adenine nucleotide, thymine nucleotide, guanine nucleotide, and cytosine nucleotide, respectively.

**Table 3: Nucleotide Mixture Solution 2**

| Reagent name | Final concentration (nmol/L) |
|---|---|
| Cold dATP | 200 |
| Cold dTTP | 200 |
| Cold dGTP | 200 |
| Cold dCTP | 200 |

Among them, Cold dATP referred to adenine nucleotide modified only with reversible blocking group, Cold dTTP referred to thymine nucleotide modified only with reversible blocking group, Cold dGTP referred to guanine nucleotide modified only with reversible blocking group, and Cold dCTP referred to cytosine nucleotide modified only with reversible blocking group.

### 3) Preparation of Nucleotide Mixture Solution 3

As shown in Table 4 below, all nucleotides in Nucleotide Mixture Solution 3 are dideoxynucleotides, in which ddATP referred to adenine triphosphate dideoxynucleotide, ddTTP referred to thymine triphosphate dideoxynucleotide, ddGTP referred to guanine triphosphate dideoxynucleotide, and ddCTP referred to cytosine triphosphate dideoxynucleotide.

**Table 4: Nucleotide Mixture Solution 3**

| Reagent name | Final concentration (nmol/L) |
|---|---|
| ddATP | 200 |
| ddTTP | 200 |
| ddGTP | 200 |
| ddTTP | 200 |

### 4) Preparation of Synthesis Reagent 1

Synthesis Reagent 1 was prepared according to Table 5 below by mixing for later use. The nucleotides of Synthesis Reagent 1 were nucleotides modified with both fluorescent dye and reversible blocking group.

**Table 5: Synthesis Reagent 1**

| Reagent name | Dosage (ml) |
|---|---|
| Reaction buffer | 57.6 |
| Nucleotide Mixture Solution 1 | 1.2 |
| DNA polymerase | 1.2 |

### 5) Preparation of Synthesis Reagent 2

Synthesis Reagent 2 was prepared according to Table 6 below by mixing for later use. The nucleotides of Synthesis Reagent 2 were nucleotides modified only with reversible blocking group.

**Table 6: Synthesis Reagent 2**

| Reagent name | Dosage (ml) |
|---|---|
| Reaction buffer | 57.6 |
| Nucleotide Mixture Solution 2 | 1.2 |
| DNA polymerase | 1.2 |

6) Preparation of Synthesis Reagents 3-1, 3-2, 3-3 and 3-4. These nucleotide mixture solutions were respectively prepared with Nucleotide Mixture Solution 1 and Nucleotide Mixture Solution 2 in different concentration ratios. The specific concentration ratios were shown in Tables 7 to 10.

Synthesis Reagent 3-1 was prepared according to Table 7 below by mixing for later use.

**Table 7: Synthesis Reagent 3-1**

| Reagent name | Dosage (ml) |
|---|---|
| Reaction buffer | 57.6 |
| Nucleotide Mixture Solution 1 | 0.6 |
| Nucleotide Mixture Solution 2 | 0.6 |
| DNA polymerase | 1.2 |

### 7) Preparation of Synthesis Reagent 3-2

Synthesis Reagent 3-2 was prepared according to Table 8 below by mixing for later use.

**Table 8: Synthesis Reagent 3-2**

| Reagent name | Dosage (ml) |
|---|---|
| Reaction buffer | 57.6 |
| Nucleotide Mixture Solution 1 | 0.72 |
| Nucleotide Mixture Solution 2 | 0.48 |
| DNA polymerase | 1.2 |

### 8) Preparation of Synthesis Reagent 3-3

Synthesis Reagent 3-3 was prepared according to Table 9 below by mixing for later use.

**Table 9: Synthesis Reagent 3-3**

| Reagent name | Dosage (ml) |
|---|---|
| Reaction buffer | 57.6 |
| Nucleotide Mixture Solution 1 | 0.11 |
| Nucleotide Mixture Solution 2 | 1.09 |
| DNA polymerase | 1.2 |

### 9) Preparation of Synthesis Reagent 3-4

Synthesis Reagent 3-4 was prepared according to Table 10 below by mixing for later use.

**Table 10: Synthesis Reagent 3-4**

| Reagent name | Dosage (ml) |
|---|---|
| Reaction buffer | 57.6 |
| Nucleotide Mixture Solution 1 | 1.14 |
| Nucleotide Mixture Solution 2 | 0.06 |
| DNA polymerase | 1.2 |

### 10) Preparation of Synthesis Reagent 4

Synthesis Reagent 4 was prepared according to Table 11 below by mixing for later use.

**Table 11: Synthesis Reagent 4**

| Reagent name | Dosage (ml) |
|---|---|
| Reaction buffer | 57.6 |
| Nucleotide Mixture Solution 3 | 1.2 |
| DNA polymerase | 1.2 |

11) Preparation of Synthesis Reagents 5-1, 5-2 and 5-3. These nucleotide mixture solutions were respectively prepared by mixing Nucleotide Mixture Solution 2 and Nucleotide Mixture Solution 3 in different concentration ratios. The specific concentration ratios were shown in Tables 12 to 14.

Synthesis Reagent 5-1 was prepared according to Table 12 below by mixing for later use.

**Table 12: Synthesis Reagent 5-1**

| Reagent name | Dosage (ml) |
|---|---|
| Reaction buffer | 57.6 |
| Nucleotide Mixture Solution 2 | 0.12 |
| Nucleotide Mixture Solution 3 | 12 |
| DNA polymerase | 1.2 |

### 12) Preparation of Synthesis Reagent 5-2

Synthesis Reagent 5-2 was prepared according to Table 13 below by mixing for later use.

**Table 13: Synthesis Reagent 5-2**

| Reagent name | Dosage (ml) |
|---|---|
| Reaction buffer | 57.6 |
| Nucleotide Mixture Solution 2 | 12 |
| Nucleotide Mixture Solution 3 | 0.12 |
| DNA polymerase | 1.2 |

### 13) Preparation of Synthesis Reagent 5-3

Synthesis Reagent 5-3 was prepared according to Table 14 below by mixing for later use.

**Table 14: Synthesis Reagent 5-3**

| Reagent name | Dosage (ml) |
|---|---|
| Reaction buffer | 57.6 |
| Nucleotide Mixture Solution 2 | 12 |
| Nucleotide Mixture Solution 3 | 0.24 |
| DNA polymerase | 1.2 |

### Example 2

In all the following experiments, *E. coli* single-stranded circular DNA (i.e., MGI's standard library reagent V3.0) was used as a template, the MGISEQ-2000RS high-throughput sequencing kit (MGI) was used to complete the preparation of DNA nanospheres that were loaded into the chip for subsequent sequencing.

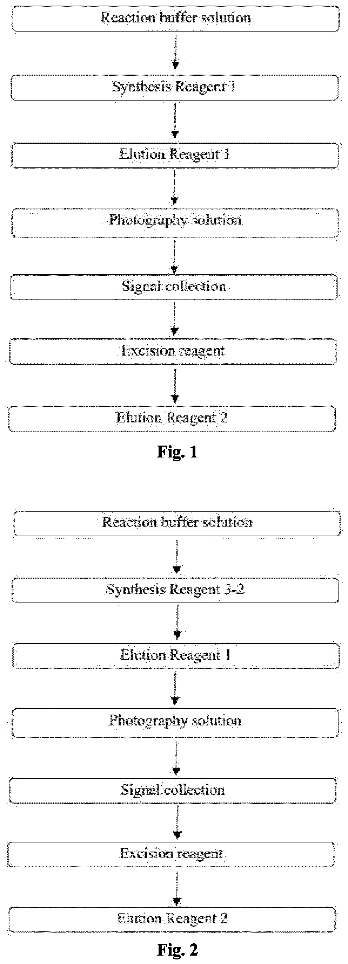

Experimental Group 1: The MGISEQ-2000RS high-throughput sequencing kit was used, the #1 well reagent in the kit was removed and replaced with Synthesis Reagent 1 prepared above, that was, a reaction solution of nucleotide mixture solution with both fluorescence and reversible blocking modification group. For each cycle of reaction, only one round of polymerization was performed, and the polymerized nucleotides were a nucleotide mixture solution with both fluorescence and reversible blocking modification group. The MGISEQ-2000RS sequencing platform was used to perform SE100 sequencing according to the experimental process as shown in Fig. 1. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 2: The MGISEQ-2000RS high-throughput sequencing kit was used, the #1 well reagent in the kit was removed and replaced with Synthesis Reagent 3-2 prepared above, that was, a reaction solution prepared with Nucleotide Mixture Solution 1 with both fluorescence and reversible blocking modification group into which Nucleotide Mixture Solution 2 with only reversible blocking was incorporated in ratio of 3:2. For each cycle of reaction, only one round of polymerization was performed, and the polymerized nucleotides were a mixture prepared with Nucleotide Mixture Solution 1 and Nucleotide Mixture Solution 2 in ratio of 3:2. The MGISEQ-2000RS sequencing platform was used to perform SE100 sequencing according to the experimental process as shown in Fig. 2. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

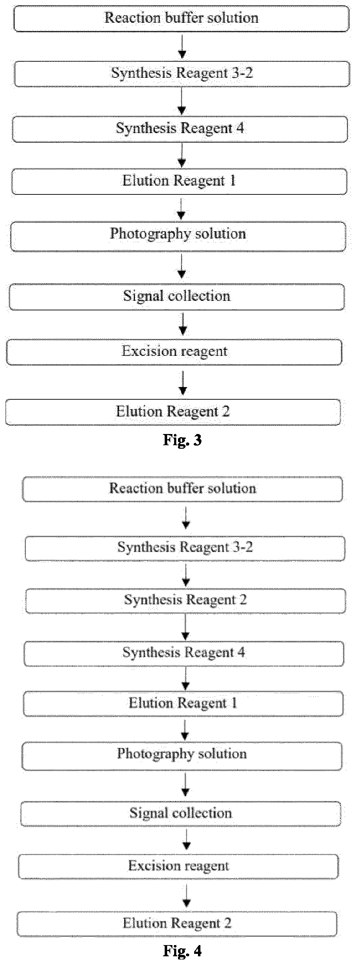

Experimental Group 3: The MGISEQ-2000RS high-throughput sequencing kit was used, the #1 well reagent and the #2 well reagent in the kit were removed, in which the #1 well reagent was replaced with Synthesis Reagent 3-2 of the sequencing group, the #2 well reagent was replaced with Synthesis Reagent 4 of the sequencing group, 2 rounds of polymerization were performed for each cycle of reaction, the polymerized nucleotides in the first round were a mixture prepared with Nucleotide Mixture Solution 1 and Nucleotide Mixture Solution 2 in ratio of 3:2, and those in the second round were the dideoxynucleotide mixture solution. The MGISEQ-2000RS sequencing platform was used to perform SE100 sequencing according to the experimental process as shown in Fig. 3. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 4: The MGISEQ-2000RS high-throughput sequencing kit was used, the #1 well reagent, the #2 well reagent and the #17 well reagent in the kit were removed, in which the #1 well reagent was replaced with Synthesis Reagent 3-2 of the sequencing group, the #2 well reagent was replaced with Synthesis Reagent 2 of the sequencing group, and the #17 well reagent was replaced with Synthesis Reagent 4 of the sequencing group. The MGISEQ-2000RS sequencing platform was used to perform SE100 sequencing according to the experimental process as shown in Fig. 4, in which 3 rounds of polymerization were performed for each cycle of reaction, the polymerized nucleotides in the first round were a mixture prepared with Nucleotide Mixture Solution 1 and Nucleotide Mixture Solution 2 in ratio of 3:2, those in the second round were Nucleotide Mixture Solution 2, and those in the third round were the dideoxynucleotide mixture solution. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

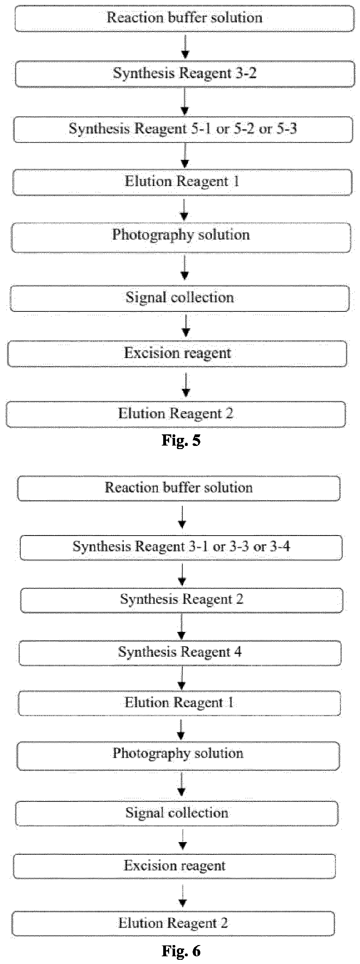

Experimental Group 5: The MGISEQ-2000RS high-throughput sequencing kit was used, the #1 well reagent and the #2 well reagent in the kit were removed, in which the #1 well reagent was replaced with Synthesis Reagent 3-2 of the sequencing group, the #2 well reagent was replaced with Synthesis Reagent 5-1 of the sequencing group, 2 rounds of polymerization were performed for each cycle of reaction, the polymerized nucleotides in the first round were a mixture prepared with Nucleotide Mixture Solution 1 and Nucleotide Mixture Solution 2 in ratio of 3:2, and those in the second round were a mixture solution prepared with Nucleotide Mixture Solution 2 and the dideoxynucleotide mixture solution in ratio of 1:100. The MGISEQ-2000RS sequencing platform was used to perform SE100 sequencing according to the experimental process as shown in Fig. 5. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 6: The MGISEQ-2000RS high-throughput sequencing kit was used, the #1 well reagent and the #2 well reagent in the kit were removed, in which the #1 well reagent was replaced with Synthesis Reagent 3-2 of the sequencing group, the #2 well reagent was replaced with Synthesis Reagent 5-2 of the sequencing group, 2 rounds of polymerization were performed for each cycle of reaction, the polymerized nucleotides in the first round were a mixture prepared with Nucleotide Mixture Solution 1 and Nucleotide Mixture Solution 2 in ratio of 3:2, and those in the second round were a mixture solution prepared with Nucleotide Mixture Solution 2 and the dideoxynucleotide mixture solution in ratio of 100:1. The MGISEQ-2000RS sequencing platform was used to perform SE100 sequencing according to the experimental process as shown in Fig. 5. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 7: The MGISEQ-2000RS high-throughput sequencing kit was used, the #1 well reagent and the #2 well reagent in the kit were removed, in which the #1 well reagent was replaced with Synthesis Reagent 3-2 of the sequencing group, the #2 well reagent was replaced with Synthesis Reagent 5-3 of the sequencing group, 2 rounds of polymerization were performed for each cycle of reaction, the polymerized nucleotides in the first round were a mixture prepared with Nucleotide Mixture Solution 1 and Nucleotide Mixture Solution 2 in ratio of 3:2, and those in the second round were a mixture solution prepared with Nucleotide Mixture Solution 2 and the dideoxynucleotide mixture solution in ratio of 50:1. The MGISEQ-2000RS sequencing platform was used to perform SE100 sequencing according to the experimental process as shown in Fig. 5. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 8: The MGISEQ-2000RS high-throughput sequencing kit was used, the #1 well reagent, the #2 well reagent and the #17 well reagent in the kit were removed, in which the #1 well reagent was replaced with Synthesis Reagent 3-1 of the sequencing group, the #2 well reagent was replaced with Synthesis Reagent 2 of the sequencing group, and the #17 well reagent was replaced with Synthesis Reagent 4 of the sequencing group. The MGISEQ-2000RS sequencing platform was used to perform SE100 sequencing according to the experimental process as shown in Fig. 6, in which 3 rounds of polymerization were performed for each cycle of reaction, the polymerized nucleotides in the first round were a mixture prepared with Nucleotide Mixture Solution 1 and Nucleotide Mixture Solution 2 in ratio of 1:1, those in the second round were Nucleotide Mixture Solution 2, and those in the third round were the dideoxynucleotide mixture solution. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 9: The MGISEQ-2000RS high-throughput sequencing kit was used, the #1 well reagent, the #2 well reagent and the #17 well reagent in the kit were removed, in which the #1 well reagent was replaced with Synthesis Reagent 3-3 of the sequencing group, the #2 well reagent was replaced with Synthesis Reagent 2 of the sequencing group, and the #17 well reagent was replaced with Synthesis Reagent 4 of the sequencing group. The MGISEQ-2000RS sequencing platform was used to perform SE100 sequencing according to the experimental process as shown in Fig. 6, in which 3 rounds of polymerization were performed for each cycle of reaction, the polymerized nucleotides in the first round were a mixture prepared with Nucleotide Mixture Solution 1 and Nucleotide Mixture Solution 2 in ratio of 1:10, those in the second round were Nucleotide Mixture Solution 2, and those in the third round were the dideoxynucleotide mixture solution. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

Experimental Group 10: The MGISEQ-2000RS high-throughput sequencing kit was used, the #1 well reagent, the #2 well reagent and the #17 well reagent in the kit were removed, in which the #1 well reagent was replaced with Synthesis Reagent 3-4 of the sequencing group, the #2 well reagent was replaced with Synthesis Reagent 2 of the sequencing group, and the #17 well reagent was replaced with Synthesis Reagent 4 of the sequencing group. The MGISEQ-2000RS sequencing platform was used to perform SE100 sequencing according to the experimental process as shown in Fig. 6, in which 3 rounds of polymerization were performed for each cycle of reaction, the polymerized nucleotides in the first round were a mixture prepared with Nucleotide Mixture Solution 1 and Nucleotide Mixture Solution 2 in ratio of 20:1, those in the second round were Nucleotide Mixture Solution 2, and those in the third round were the dideoxynucleotide mixture solution. In short, by following the instructions of the MGISEQ-2000RS high-throughput sequencing kit, the elution reagent was used to elute free nucleotides, the photography reagent was used to collect signals, the excision reagent was used to remove protective group, and the elution reagent was used for washing, successively. Then, the Q30 decline for each cycle and the sequencing error rate curve for each cycle were calculated to evaluate the sequencing quality.

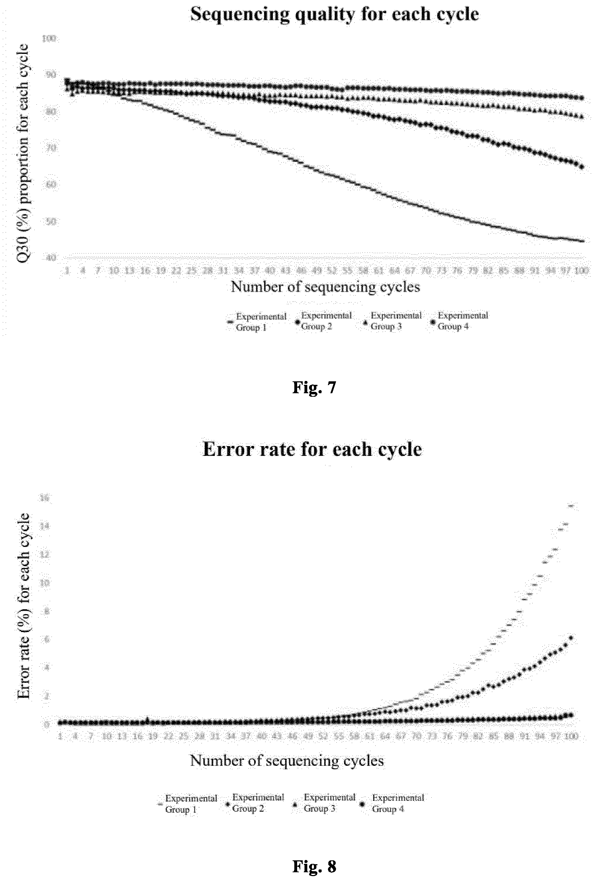

Results: As shown in Fig. 7 to 12 below, Fig. 7 shows the Q30 (%) proportion results for each cycle of four experiments, i.e., Experimental Groups 1 to 4. Among them, the abscissa is the number of sequencing cycles, and the ordinate is the Q30 (%) proportion for each cycle.

Fig. 8 shows the sequencing error rate (%) results for each cycle of four experiments, i.e., Experimental Groups 1 to 4. Among them, the abscissa is the number of sequencing cycles, and the ordinate is the sequencing error rate (%) for each cycle.

Fig. 9 shows the results of five experiments, i.e., Experimental Groups 2, 3, 5 to 7. Taking Experiment Group 2 as the control, in all other experiments, 2 rounds of polymerization were performed for each cycle of reaction, in which the nucleotides polymerized in the first round were a mixture prepared with Nucleotide Mixture Solution 1 and Nucleotide Mixture Solution 2 in ratio of 3:2, and those in the second round were mixtures of Nucleotide Mixture Solution 2 and the dideoxynucleotide mixture solution in different ratios. Among them, the abscissa is the number of sequencing cycles, and the ordinate is the Q30 (%) proportion for each cycle.

Fig. 10 shows the results of five experiments, i.e., Experimental Groups 2, 3, 5 to 7. Taking Experiment Group 2 as the control, in all other experiments, 2 rounds of polymerization were performed for each cycle of reaction, in which the nucleotides polymerized in the first round were a mixture prepared with Nucleotide Mixture Solution 1 and Nucleotide Mixture Solution 2 in ratio of 3:2, and those in the second round were mixtures of Nucleotide Mixture Solution 2 and dideoxynucleotide mixture in different ratios. Among them, the abscissa is the number of sequencing cycles, and the ordinate is the sequencing error rate (%) for each cycle.

Fig. 11 shows the results of five experiments, i.e., Experimental Groups 2, 4, 8 to 10. Taking Experiment Group 2 as the control, in all other experiments, 3 rounds of polymerization were performed for each cycle of reaction, in which the nucleotides polymerized in the first round were a mixture prepared with Nucleotide Mixture Solution 1 and Nucleotide Mixture Solution 2 in different concentration ratios, those in the second round were Nucleotide Mixture Solution 2, and those in the third round were the dideoxynucleotide mixture. Among them, the abscissa is the number of sequencing cycles, and the ordinate is the Q30 (%) proportion for each cycle.

Fig. 12 shows the results of five experiments, i.e., Experimental Groups 2, 4, 8 to 10. Taking Experiment Group 2 as the control, in all other experiments, 3 rounds of polymerization were performed for each cycle of reaction, in which the nucleotides polymerized in the first round were a mixture prepared with Nucleotide Mixture Solution 1 and Nucleotide Mixture Solution 2 in different concentration ratios, those in the second round were Nucleotide Mixture Solution 2, and those in the third round were the dideoxynucleotide mixture. Among them, the abscissa is the number of sequencing cycles, and the ordinate is the sequencing error rate (%) for each cycle.

The higher the proportion of Q30 (%) in each cycle, the better the quality; and the lower the Q30 decline, the better the quality. The lower the sequencing error rate (%) for each cycle, the better the sequencing quality.

The results in Fig. 7 and 8 showed that the sequencing quality and error rate of Experimental Group 4 were the best, followed by Experimental Group 3, and the results of both of them were significantly better than those of Experimental Group 2 and Experimental Group 1. It was confirmed that the polymerization method of the present application was better than the existing polymerization methods. Based on Fig. 9 and 10, Experimental Groups 3, 5 to 7 were significantly better than Experimental Group 2 (control), while Experimental Group 6 had the best results (it had the smallest Q30 decline). It was confirmed that the effect of the two-round polymerization method of the present application was better than that of the single-round polymerization.

Based on Fig. 11 and 12, Experimental Groups 4, 8, 9, and 10 were all better than Experimental Group 2 (control group), while Experimental Group 4 was the best. It was confirmed that the effect of the three-round polymerization method of the present application was better than that of the single-round polymerization.

## Claims

1. A method for analyzing a sequence of target polynucleotide, comprising
(a) providing a target polynucleotide,
(b) contacting the target polynucleotide with a primer under a condition that allows hybridization or annealing, thereby forming a partial duplex comprising the target polynucleotide and the primer used as a growing chain,
(c) contacting the partial duplex with a polymerase and a first nucleotide mixture under a condition that allows the polymerase to perform a nucleotide polymerization reaction such that the growing chain is extended, wherein the first nucleotide mixture comprises at least one nucleotide labeled with a label;
optionally, the first nucleotide mixture further comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
wherein each nucleotide in the first nucleotide mixture comprises on its ribose or deoxyribose moiety a protecting group capable of reversibly blocking nucleic acid chain extension (e.g., a protecting group attached via a 2'- or 3'-oxygen atom group);
(d) contacting the product of the previous step with a polymerase and a second nucleotide mixture under a condition that allows the polymerase to perform a nucleotide polymerization reaction such that the growing chain is extended, wherein the second nucleotide mixture comprises at least one (e.g., one, two, three, or four) of: (1) an unlabeled nucleotide, or (2) an irreversible blocking nucleotide, or (3) a combination of the unlabeled nucleotide and irreversible blocking nucleotide;
preferably, the second nucleotide mixture comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
wherein each unlabeled nucleotide in the second nucleotide mixture comprises on its ribose or deoxyribose moiety a protecting group capable of reversibly blocking nucleotide chain extension (e.g., a protecting group attached via a 2'- or 3'-oxygen atom);
(e) not performing this step if at least one nucleotide in the second nucleotide mixture comprises an irreversible blocking nucleotide;
contacting the product of the previous step with a polymerase and a third nucleotide mixture under a condition that allows the polymerase to perform a nucleotide polymerization reaction such that the growing chain is extended if the second nucleotide mixture does not comprise an irreversible blocking nucleotide; wherein the third nucleotide mixture comprises at least one irreversible blocking nucleotide;
(f) detecting the presence of a label in the product of the previous step;
(g) removing the protective group and label contained in the product of the previous step;
(h) optionally repeating steps (c) to (g) one or more times;
thereby, obtaining a sequence information of the target polynucleotide.

2. The method according to claim 1, wherein in step (c), the extension is an extension using the target polynucleotide as a template; preferably, the extension is an extension of one nucleotide;
preferably, the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide; optionally, the first nucleotide mixture further comprises at least one unlabeled nucleotides; or
preferably, the first nucleotide mixture comprises a first nucleotide labeled with a first label, a second nucleotide labeled with a second label, a third nucleotide labeled with a third label, a fourth nucleotide labeled with a fourth label, an unlabeled first nucleotide, an unlabeled second nucleotide, an unlabeled third nucleotide, and an unlabeled fourth nucleotide, or comprises a first nucleotide labeled with a first label, a second nucleotide labeled with a second label, a third nucleotide co-labeled with the first label and the second label, an unlabeled fourth nucleotide, an unlabeled first nucleotide, an unlabeled second nucleotide and an unlabeled third nucleotide;
preferably, the ratio of the first nucleotide labeled with the first label to the unlabeled first nucleotide is 20:1 to 1:10 (e.g., 10:1 to 1:10, 5:1 to 1:5, 3:1 to 1:3); more preferably, the ratio of the first nucleotide labeled with the first label to the unlabeled first nucleotide is 20:1, 1:10, 1:1 or 3:2; most preferably, the ratio of the first nucleotide labeled with the first label to the unlabeled first nucleotide is 3:2;
preferably, the ratio of the second nucleotide labeled with the second label to the unlabeled second nucleotide is 20:1 to 1:10 (e.g., 10:1 to 1:10, 5:1 to 1:5, 3:1 to 1:3); more preferably, the ratio of the second nucleotide labeled with the second label to the unlabeled second nucleotide is 20:1, 1:10, 1:1 or 3:2; most preferably, the ratio of the second nucleotide labeled with the second label to the unlabeled second nucleotide is 3:2;
preferably, the ratio of the third nucleotide labeled with the third label to the unlabeled third nucleotide is 20:1 to 1:10 (e.g., 10:1 to 1:10, 5:1 to 1:5, 3:1 to 1:3); more preferably, the ratio of the third nucleotide labeled with the third label to the unlabeled third nucleotide is 20:1, 1:10, 1:1 or 3:2; most preferably, the ratio of the third nucleotide labeled with the third label to the unlabeled third nucleotide is 3:2;
preferably, the ratio of the third nucleotide co-labeled with the first label and the second label to the unlabeled third nucleotide is 20:1 to 1:10 (e.g., 10:1 to 1:10, 5:1 to 1:5, 3:1 to 1:3); more preferably, the ratio of the third nucleotide co-labeled with the first label and the second label to the unlabeled third nucleotide is 20:1, 1:10, 1:1 or 3:2; most preferably, the ratio of the third nucleotide co-labeled with the first label and the second label to the unlabeled third nucleotides is 3:2;
preferably, the ratio of the fourth nucleotide labeled with the fourth label to the unlabeled fourth nucleotide is 20:1 to 1:10 (e.g., 10:1 to 1:10, 5:1 to 1:5, 3:1 to 1:3); more preferably, the ratio of the fourth nucleotide labeled with the fourth label to the unlabeled fourth nucleotide is 20:1, 1:10, 1:1 or 3:2; most preferably, the ratio of the fourth nucleotide labeled with the fourth label to the unlabeled fourth nucleotide is 3:2.

3. The method according to claim 1 or 2, wherein, in step (d), the second nucleotide mixture comprises:
(1) an unlabeled first nucleotide, an unlabeled second nucleotide, an unlabeled third nucleotide, and an unlabeled fourth nucleotide; or,
(2) a first irreversible blocking nucleotide, a second irreversible blocking nucleotide, a third irreversible blocking nucleotide, and a fourth irreversible blocking nucleotide; or,
(3) an unlabeled first nucleotide, an unlabeled second nucleotide, an unlabeled third nucleotide, an unlabeled fourth nucleotide, and a first irreversible blocking nucleotide, a second irreversible blocking nucleotide, a third irreversible blocking nucleotide, and a fourth irreversible blocking nucleotide;
preferably, the ratio of the unlabeled first nucleotide to the first irreversible blocking nucleotide is 100:1 to 1:100; more preferably, the ratio of the unlabeled first nucleotide to the first irreversible blocking nucleotide is 1:100,50:1 or 100:1; most preferably, the ratio of the unlabeled first nucleotide to the first irreversible blocking nucleotide is 100:1;
preferably, the ratio of the unlabeled second nucleotide to the second irreversible blocking nucleotide is 100:1 to 1:100; more preferably, the ratio of the unlabeled second nucleotide to the second irreversible blocking nucleotide is 1:100, 50:1 or 100:1; most preferably, the ratio of the unlabeled second nucleotide to the second irreversible blocking nucleotide is 100:1;
preferably, the ratio of the unlabeled third nucleotide to the third irreversible blocking nucleotide is 100:1 to 1:100; more preferably, the ratio of the unlabeled third nucleotide to the third irreversible blocking nucleotide is 1:100, 50:1 or 100:1; most preferably, the ratio of the unlabeled third nucleotide to the third irreversible blocking nucleotide is 100:1;
preferably, the ratio of the unlabeled fourth nucleotide to the fourth irreversible blocking nucleotide is 100:1 to 1:100; more preferably, the ratio of the unlabeled fourth nucleotide to the fourth irreversible blocking nucleotide is 1:100, 50:1 or 100:1; most preferably, the ratio of the unlabeled fourth nucleotide to the fourth irreversible blocking nucleotide is 100:1.

4. The method according to any one of claims 1 to 3, wherein the first label, the second label, the third label and the fourth label are each independently the same or different;
preferably, the first label, the second label, the third label and the fourth label are different;
preferably, the first label, the second label, the third label and the fourth label are luminescent labels (e.g., fluorescent labels);
more preferably, the first label, the second label, the third label and the fourth label are each independently selected from the group consisting of coumarin, AlexaFluor, Bodipy, fluorescein, tetramethylrhodamine, phenoxazine, acridine, Cy5, Cy3, AF532, Texas Red and derivative thereof;
preferably, the target polynucleotide comprises or is DNA, RNA, or any combination thereof; preferably, the extension product of the nucleic acid molecule is DNA;
preferably, the target polynucleotide is obtained from a sample derived from eukaryote (e.g., animal, plant, fungus), prokaryote (e.g., bacterium, actinomycete), virus, phage, or any combination thereof;
preferably, the first nucleotide, the second nucleotide, the third nucleotide and the fourth nucleotide are each independently selected from the group consisting of A, T, C, G, U;
preferably, the first nucleotide, the second nucleotide, the third nucleotide and the fourth nucleotide are different;
further preferably, the first nucleotide, the second nucleotide, the third nucleotide and the fourth nucleotide are A, T, C, G, respectively;
preferably, the first irreversible blocking nucleotide, the second irreversible blocking nucleotide, the third irreversible blocking nucleotide and the fourth irreversible blocking nucleotide are each independently selected from the group consisting of A, T, C, G, U;
preferably, the first irreversible blocking nucleotide, the second irreversible blocking nucleotide, the third irreversible blocking nucleotide and the fourth irreversible blocking nucleotide are different;
further preferably, the first irreversible blocking nucleotide, the second irreversible blocking nucleotide, the third irreversible blocking nucleotide and the fourth irreversible blocking nucleotide are A, T, C, G, respectively;
preferably, the irreversible blocking nucleotide is a dideoxynucleotide;
preferably,
when the first nucleotide mixture comprises a first nucleotide labeled with a first label, a second nucleotide labeled with a second label, a third nucleotide labeled with a third label, and a fourth nucleotide labeled with a fourth label; the second nucleotide mixture comprises an unlabeled first nucleotide, an unlabeled second nucleotide, an unlabeled third nucleotide, and an unlabeled fourth nucleotide; or
when the first nucleotide mixture comprises a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label and an unlabeled fourth nucleotide, the second nucleotide mixture comprises an unlabeled first nucleotide, an unlabeled second nucleotide, and an unlabeled third nucleoside.

5. A kit, which comprises:
(a) a first nucleotide mixture, which comprises at least one nucleotide labeled with a label;
optionally, the first nucleotide mixture further comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
wherein each nucleotide in the first nucleotide mixture comprises on its ribose or deoxyribose moiety a protecting group capable of reversibly blocking nucleic acid chain extension (e.g., a protecting group attached via a 2'- or 3'-oxygen atom);
(b) a second nucleotide mixture, which comprises at least one (e.g., one, two, three or four) of (1) an unlabeled nucleotide, or (2) an irreversible blocking nucleotide, or (3) a combination of the unlabeled nucleotide and irreversible blocking nucleotide;
preferably, the second nucleotide mixture comprises at least one unlabeled nucleotide; for example, comprises a nucleotide selected from the group consisting of: an unlabeled first nucleotide, an unlabeled second nucleotide, an unlabeled third nucleotide, an unlabeled fourth nucleotide, or any combination thereof;
wherein each unlabeled nucleotide in the second nucleotide mixture comprises on its ribose or deoxyribose moiety a protecting group capable of reversibly blocking nucleic acid chain extension (e.g., a protecting group attached via a 2'- or 3'-oxygen atom);
(c) if the second nucleotide mixture does not comprise an irreversible blocking nucleotide, the kit comprises a third nucleotide mixture which comprises at least one irreversibly blocking nucleotide;
if at least one nucleotide in the second nucleotide mixture comprises an irreversible blocking nucleotide, the kit does not comprise a third nucleotide mixture;
preferably, the irreversible blocking nucleotide is a dideoxynucleotide.

6. The kit according to claim 5, wherein the first nucleotide mixture comprises a first nucleotide labeled with a first label, a second nucleotide labeled with a second label, a third nucleotide labeled with a third label, and a fourth nucleotide labeled with a fourth label or an unlabeled fourth nucleotide, or comprises a first nucleotide labeled with a first label, a second nucleotide labeled with a second label, a third nucleotide co-labeled with the first label and the second label, and an unlabeled fourth nucleotide; optionally, the first nucleotide mixture further comprises at least one unlabeled nucleotide; or
preferably, the first nucleotide mixture comprises a first nucleotide labeled with a first label, a second nucleotide labeled with a second label, a third nucleotide labeled with a third label, a fourth nucleotide labeled with a fourth label, an unlabeled first nucleotide, an unlabeled second nucleotide, an unlabeled third nucleotide, and an unlabeled fourth nucleotide, or comprises a first nucleotide labeled with a first label, a second nucleotide labeled with a second label, a third nucleotide co-labeled with the first label and the second label, an unlabeled fourth nucleotide, an unlabeled first nucleotide, an unlabeled second nucleotide and an unlabeled third nucleotide;
preferably, the ratio of the first nucleotide labeled with the first label to the unlabeled first nucleotide is 20:1 to 1:10 (e.g., 10:1 to 1:10, 5:1 to 1:5, 3:1 to 1:3); more preferably, the ratio of the first nucleotide labeled with the first label to the unlabeled first nucleotide is 20:1, 1:10, 1:1 or 3:2; most preferably, the ratio of the first nucleotide labeled with the first label to the unlabeled first nucleotide is 3:2;
preferably, the ratio of the second nucleotide labeled with the second label to the unlabeled second nucleotide is 20:1 to 1:10 (e.g., 10:1 to 1:10, 5:1 to 1:5, 3:1 to 1:3); more preferably, the ratio of the second nucleotide labeled with the second label to the unlabeled second nucleotide is 20:1, 1:10, 1:1 or 3:2; most preferably, the ratio of the second nucleotide labeled with the second label to the unlabeled second nucleotide is 3:2;
preferably, the ratio of the third nucleotide labeled with the third label to the unlabeled third nucleotide is 20:1 to 1:10 (e.g., 10:1 to 1:10, 5:1 to 1:5, 3:1 to 1:3); more preferably, the ratio of the third nucleotide labeled with the third label to the unlabeled third nucleotide is 20:1, 1:10, 1:1 or 3:2; most preferably, the ratio of the third nucleotide labeled with the third label to the unlabeled third nucleotide is 3:2;
preferably, the ratio of the third nucleotide co-labeled with the first label and the second label to the unlabeled third nucleotide is 20:1 to 1:10 (e.g., 10:1 to 1:10, 5:1 to 1:5, 3:1 to 1:3); more preferably, the ratio of the third nucleotide co-labeled with the first label and the second label to the unlabeled third nucleotide is 20:1, 1:10, 1:1 or 3:2; most preferably, the ratio of the third nucleotide co-labeled with the first label and the second label to the unlabeled third nucleotides is 3:2;
preferably, the ratio of the fourth nucleotide labeled with the fourth label to the unlabeled fourth nucleotide is 20:1 to 1:10 (e.g., 10:1 to 1:10, 5:1 to 1:5, 3:1 to 1:3); more preferably, the ratio of the fourth nucleotide labeled with the fourth label to the unlabeled fourth nucleotide is 20:1, 1:10, 1:1 or 3:2; most preferably, the ratio of the fourth nucleotide labeled with the fourth label to the unlabeled fourth nucleotide is 3:2.

7. The kit according to claim 5 or 6, wherein when the first nucleotide mixture comprises a first nucleotide labeled with a first label, a second nucleotide labeled with a second label, a third nucleotide labeled with a third label and a fourth nucleotide labeled with a fourth label; the second nucleotide mixture comprises an unlabeled first nucleotide, an unlabeled second nucleotide, an unlabeled third nucleotide, and an unlabeled fourth nucleotide; or
when the first nucleotide mixture comprises a first nucleotide labeled with a first label, a second nucleotide labeled with a second label, a third nucleotide labeled with a third label, and an unlabeled fourth nucleotide, or comprises a first nucleotide labeled with a first label, a second nucleotide labeled with a second label, a third nucleotide co-labeled with the first label and the second label and an unlabeled fourth nucleotide, the second nucleotide mixture comprises an unlabeled first nucleotide, an unlabeled second nucleotide, and an unlabeled third nucleoside.

8. The kit according to any one of claims 5 to 7, wherein:
the second nucleotide mixture comprises:
(1) an unlabeled first nucleotide, an unlabeled second nucleotide, an unlabeled third nucleotide, and an unlabeled fourth nucleotide; or,
(2) a first irreversible blocking nucleotide, a second irreversible blocking nucleotide, a third irreversible blocking nucleotide, and a fourth irreversible blocking nucleotide; or,
(3) an unlabeled first nucleotide, an unlabeled second nucleotide, an unlabeled third nucleotide, an unlabeled fourth nucleotide, a first irreversible blocking nucleotide, a second irreversible blocking nucleotide, a third irreversible blocking nucleotide, and a fourth irreversible blocking nucleotide;
preferably, the ratio of the unlabeled first nucleotide to the first irreversible blocking nucleotide is 100:1 to 1:100; more preferably, the ratio of the unlabeled first nucleotide to the first irreversible blocking nucleotide is 1:100, 50:1 or 100:1; most preferably, the ratio of the unlabeled first nucleotide to the first irreversible blocking nucleotide is 100:1;
preferably, the ratio of the unlabeled second nucleotide to the second irreversible blocking nucleotide is 100:1 to 1:100; more preferably, the ratio of the unlabeled second nucleotide to the second irreversible blocking nucleotide is 1:100, 50:1 or 100:1; most preferably, the ratio of the unlabeled second nucleotide to the second irreversible blocking nucleotide is 100:1;
preferably, the ratio of the unlabeled third nucleotide to the third irreversible blocking nucleotide is 100:1 to 1:100; more preferably, the ratio of the unlabeled third nucleotide to the third irreversible blocking nucleotide is 1:100, 50:1 or 100:1; most preferably, the ratio of the unlabeled third nucleotide to the third irreversible blocking nucleotide is 100:1;
preferably, the ratio of the unlabeled fourth nucleotide to the fourth irreversible blocking nucleotide is 100:1 to 1:100; more preferably, the ratio of the unlabeled fourth nucleotide to the fourth irreversible blocking nucleotide is 1:100, 50:1 or 100:1; most preferably, the ratio of the unlabeled fourth nucleotide to the fourth irreversible blocking nucleotide is 100:1;
preferably, the irreversible blocking nucleotide is a dideoxynucleotide.

9. The kit according to any one of claims 5 to 8, wherein, optionally, the kit further comprises one or more selected from the group consisting of a nucleic acid polymerase, a reagent for extension, a reagent for sequencing, or any combination thereof;
preferably, the reagent for extension comprises one or more selected from the group consisting of a primer that is fully or partially complementary to the polynucleotide, a working buffer for enzyme (e.g., nucleic acid polymerase), water, a solution containing ions (e.g., Mg²⁺), a single-stranded DNA binding protein, or any combination thereof;
preferably, the reagent for sequencing comprises one or more selected from the group consisting of a sequencing slide, a reagent for removing protective group and label on nucleotide, and a reagent for detecting luminescence signal of the label (e.g. fluorescent mixture);
preferably, the nucleic acid polymerase is a DNA polymerase, such as a thermostable DNA polymerase; preferably, the thermostable DNA polymerase is obtained from *Thermus aquaticus (Taq), Thermus thermophiles (Tth), Thermus filiformis, Thermis flavus, Thermococcus literalis, Thermus antranildanii, Thermus caldophllus, Thermus chliarophilus, Thermus flavus, Thermus igniterrae, Thermus lacteus, Thermus oshimai, Thermus ruber, Thermus rubens, Thermus scotoductus, Thermus silvanus, Thermus thermophilus, Thermotoga maritima, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus woesei, Pyrococcus horikoshii, Pyrococcus abyssi, Pyrodictium occultum, Aquifexpyrophilus* and *Aquifex aeolieus;*
preferably, the kit is used for analyzing a polynucleotide;
preferably, the kit is used for sequencing a polynucleotide;
preferably, the presence of the label is detected by a luminescent signal;
preferably, the presence of the label is detected by one or more (e.g., 2, 3, 4) kinds of luminescent signals;
preferably, the first label, the second label, the third label and the fourth label are each independently the same or different;
preferably, the first label, the second label, the third label and the fourth label are different;
preferably, the first label, the second label, the third label and the fourth label are luminescent labels (e.g., fluorescent labels);
more preferably, the first label, the second label, the third label and the fourth label are each independently selected from the group consisting of coumarin, AlexaFluor, Bodipy, fluorescein, tetramethylrhodamine, phenoxazine, Acridine, Cy5, Cy3, AF532, EF700, Texas Red and derivative thereof;
preferably, the target polynucleotide comprises or is DNA, RNA, or any combination thereof; preferably, the extension product of the nucleic acid molecule is DNA;
preferably, the target polynucleotide is obtained from a sample derived from an eukaryote (e.g., animal, plant, fungus), a prokaryote (e.g., bacterium, actinomycete), a virus, a phage, or any combination thereof;
preferably, the first nucleotide, the second nucleotide, the third nucleotide and the fourth nucleotide are each independently selected from the group consisting of A, T, C, G, U;
preferably, the first nucleotide, the second nucleotide, the third nucleotide and the fourth nucleotide are different;
further preferably, the first nucleotide, the second nucleotide, the third nucleotide and the fourth nucleotide are A, T, C and G, respectively;
preferably, the first irreversible blocking nucleotide, the second irreversible blocking nucleotide, the third irreversible blocking nucleotide and the fourth irreversible blocking nucleotide are each independently selected from the group consisting of A, T, C, G, U;
preferably, the first irreversible blocking nucleotide, the second irreversible blocking nucleotide, the third irreversible blocking nucleotide and the fourth irreversible blocking nucleotide are different;
further preferably, the first irreversible blocking nucleotide, the second irreversible blocking nucleotide, the third irreversible blocking nucleotide and the fourth irreversible blocking nucleotide are A, T, C, and G, respectively.
